# EUROPEAN PATENT APPLICATION

(11) **EP 1 944 055 A1**
(43) Date of publication of application: **16.07.2008**
(21) Application number: 06743427.4
(22) Date of filing: 07.04.2006
(51) Int. Cl.: A61M 39/22, A61M 25/10

(54) **VALVATE VESICAL CATHETER**

(30) Priority: 28.09.2005 ES 200502116 U
(71) Applicant: Rada Martinez, Ignacio, 28022 Madrid (ES)
(72) Inventor: Rada Martinez, Ignacio, 28022 Madrid (ES)
(74) Representative: Elzaburu Marquez, Alberto
(86) International application number: PCT/ES2006/000167
(87) International publication number: WO 2007/036580

(57) **Abstract**

The invention relates to a valvate vesical catheter of the type that is formed by a flexible tube of plastic material, which is intended to be introduced through the urethra into the bladder. The distal end of the aforementioned tube terminates in a fork comprising two branches, namely a first branch which is used to inflate the balloon which stabilises the catheter in the bladder and a second branch through which urine flows. The invention is **characterised in that** the second urine discharge branch includes a valve element which is stably and hermetically joined to the actual catheter, said valve element comprising means which can be actuated by the patient in order to open and close same. In addition, the inventive catheter is **characterised in that** the valve element includes a toothed neck that can be pressure-ccupled, in a tongue-and-groove manner, to the end of the urine discharge branch. The inventive catheter is also **characterised in that** the valve element is inserted hermetically into the body of the distal end of the catheter which is intended to release the urine. The invention is further **characterised in that** the valve element is of the type that comprises a lever for actuating a rotary obturator that can rotate by'90° between open and closed end positions.

## Description

### Object of the Invention

This invention refers to a valved urinary catheter, i.e. a catheter of the type that is inserted through the urethra to the bladder, to establish direct, permanent communication between the bladder and the exterior, thereby overcoming problems of urethral obstruction caused by prostatic hypertrophy.

The object of the invention is to obtain improved functional features of the catheter, from the point of view of user handling, while simultaneously minimising the risk of infections and generally improving the quality of life for this type of patient.

### BACKGROUND TO THE INVENTION

Today, there are a large number of patients who have to wear permanent urinary catheters due to different medical indications.

In most cases this is due to prostate disease which impedes adequate emptying of the urinary bladder. Prostatic adenoma or benign prostatic hypertrophy are inherent pathologies of advanced age, an epiphenomenon of ageing and thus extremely frequent in our society. The solution to this problem, which impedes normal micturition, consists of surgical resection, either through the urethra or by open surgery through an abdominal incision. However, for both this pathology and others, in many patients surgery is contraindicated, for example in geriatric patients with other associated pathologies that involve a very high surgical risk, and it is among precisely these patients that the need for a permanent urinary catheter is indicated.

The permanent urinary catheters currently on the market consist of flexible plastic tubes which are inserted through the urethra until they reach the bladder. These catheters are finished at their distal end, i.e. the end outside the urethra, by two lines, one of which is used to inflate a balloon which stabilises the catheter inside the bladder, preventing the catheter from coming out, while the other line enables the urine to flow to the exterior.

The permanent presence of the catheter functionally cancels out the sphincter, so that an opening-closing system for the catheter is essential, there currently being two solutions for this: one consists of a conical plastic plug which is fitted in the opening of the line through which the urine flows, a procedure that needs to be carried out by the patient every time the bladder is emptied, exerting the required pressure on the plug until the closure is water-tight. The other solution consists of a second plastic tube, attached to a bag, which is inserted under pressure into the catheter, so that the urine flows permanently until the bag is full, at which time it has to be replaced by another.

Urinary catheters must be replaced regularly, every one or two months depending on the material with which they are manufactured; they require permanent attention, and involve risks of infection to the extent that antibiotic protection is recommended for handling of the catheter for replacement, and its daily handling is uncomfortable for patients.

Concentrating specifically on the opening-closing systems of the abovementioned catheters, these present problems which are mainly due to the following aspects:
- There is a certain degree of difficulty in inserting/removing the plug. This problem is particularly serious in elderly people, who are the immense majority of people with permanent urinary catheters, as it is not unusual for them to have problems with sight, loss of capacity for hand movement, tremor, etc. which significantly complicates the procedure.
- The plug occasionally comes loose due to the pressure of the urine, which inevitably leads to spillage of the urine and the soiling of clothes. For this reason the plug must be inserted with force, which is sometimes not possible for an elderly person, obviously complicating subsequent removal.
- The plug can fall on the floor and get lost during the micturition procedure.
- Even if the procedure is carried out in the best possible way, reflux of a certain amount of urine to the exterior during the procedure is almost inevitable.
- The bags of urine are obviously uncomfortable to carry. They are generally placed around the leg using some form of attachment system, which means it possible that they can come loose, as well as representing an additional financial cost.

### DESCRIPTION OF THE INVENTION

The valved urinary bladder that the invention proposes solves the problems described above completely satisfactorily, in each and every aspect discussed.

More specifically, to do this the catheter, using the basic structure of any urinary catheter of this type, i.e. a flexible tubular body which at its distal ends forks into two lines, one for inflating the balloon that stabilises the catheter and the other for urine to flow through, centres the characteristics on the fact that in the latter line, through which the urine flows, instead of the abovementioned classic plug, there is a valvular element, specifically an air-tight plastic valve, preferably with a turning switch, which enables the patient to open or close the valve with a simple hand movement, specifically, a movement of a quarter of a turn, i.e. 90°, which provides the following advantages:
- Unlike the classic plug, the valve cannot come loose.
- It is extremely easy to handle and both opening and closing can be carried out by touch, i.e. without requiring visualisation.
- It shortens the micturition procedure.
- Handling is much quicker, minimising the risk of infection.
- It prevents loss of urine during the micturition procedure to a large extent.
- It also reduces to a large extent the need for changing and washing of a patient's clothes.
- As mentioned above, all these advantages represent a substantial improvement in the quality of life for these patients.

### DESCRIPTION OF THE DRAWINGS

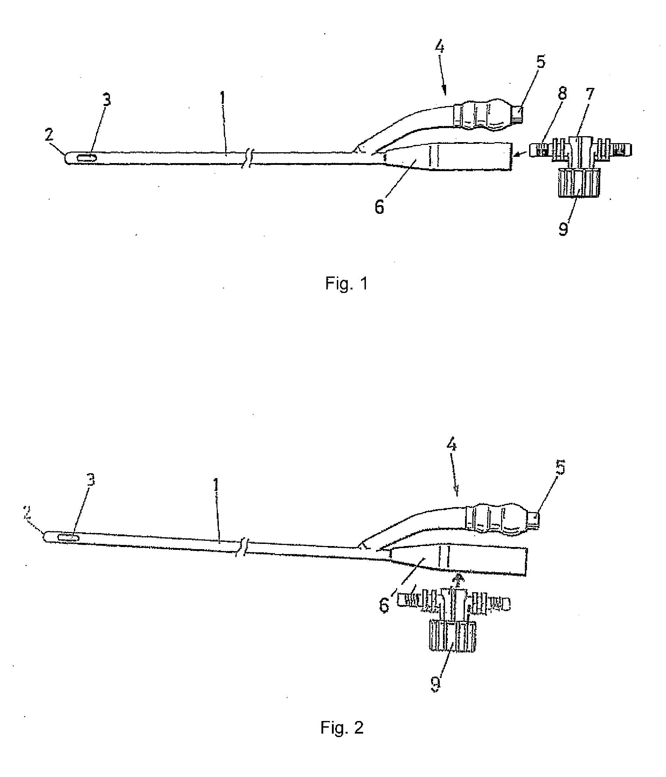

To complement the description given here and in order to help better understand the characteristics of the invention, in accordance with an example of a practical embodiment of the invention, the description is accompanied by a single sheet of illustrative and non-limiting diagrams, being an integral part of the description, and in its single figure, a valved urinary catheter made in accordance with the object of this invention has been depicted side on.

### PREFERRED EMBODIMENT OF THE INVENTION

The abovementioned figure shows how the catheter proposed by the invention is constituted, as with any conventional permanent urinary catheter, a flexible tube (1), made of appropriate plastic material, which is rounded at its proximal end (2) to facilitate insertion through the urethra and that near this proximal end there are two side holes (3), while its distal end (4) incorporates a fork into two lines (5) and (6), the former for inflating the balloon (4), while the other (6) serves to discharge the urine.

Thus, and in accordance with the invention, in the line for discharging the urine (6), a valve has been implanted (7), which is attached to the line insuring watertightness and stability in the assembly. The attachment of the valve may be fixed, inserting it a few centimetres before the distal end of the line (6) or attached to the end, for example, by means of perimeter toothing (8) of the valve body so that the valve (7) can establish a watertight seal for the urine discharge line (6) or an opening in the line.

Although the valve (7) used can be of any appropriate conventional type, it is envisaged preferentially as being fitted with a small switch or lever (9) which provides the valve seal with a 90° movement between the open and closed limit positions, so that the valve operating procedure is made as simple as possible for the patient.

## Claims

1. Valved urinary catheter, of the type structured by means of a flexible plastic tube, for inserting through the urethra until it reaches the bladder, the tube being finished at its distal end by a fork into two lines, one for inflating the balloon that stabilises the catheter and the other through which the urine flows, **characterised in that** the latter line, for releasing the urine, incorporates a valvular element, attached in a stable and air-tight way to the catheter itself, the valvular element including operating system that enables the patient to open and close it.

2. Valved urinary catheter, as per claim 1, **characterised in that** the valvular element incorporates an extended toothed neck for interlocking attachment under pressure to the end of the line used for discharging the urine.

3. Valved urinary catheter, as per claim 1, **characterised in that** the valvular element is inserted in an air-tight manner into the body of the distal end of the catheter used to release the urine.

4. Valved urinary catheter, as per the above claims, **characterised in that** the valvular element is of the type that incorporates an operating switch for a rotating seal, with a 90° turn between the valve limit positions of open and closed.
